(19) Europäisches Patentamt / European Patent Office / Office européen des brevets

(11) **EP 4 376 015 A1**

(12) **EUROPEAN PATENT APPLICATION**

(43) Date of publication:
**29.05.2024 Bulletin 2024/22**

(21) Application number: **23206115.0**

(22) Date of filing: **26.10.2023**

(51) International Patent Classification (IPC):
**G16H 20/17** (2018.01)   **G06N 20/00** (2019.01)
**G16H 40/60** (2018.01)   **G16H 40/63** (2018.01)
**G16H 40/67** (2018.01)   **G16H 50/70** (2018.01)

(52) Cooperative Patent Classification (CPC):
**G16H 20/17; G06N 20/00; G16H 40/60;
G16H 40/63; G16H 40/67; G16H 50/70**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.11.2022 FR 2212201**

(71) Applicant: **Diabeloop
38000 Grenoble (FR)**

(72) Inventors:
• **LOUIS, Maxime
38100 Grenoble (FR)**
• **ROMERO-UGALDE, Hector
38420 LE VERSOUD (FR)**

(74) Representative: **Pellegri, Michel Pascal Romain
Cabinet Vulpelex
52 Rue Montgolfier
69006 Lyon (FR)**

(54) **DEVICE AND METHOD FOR DETERMINING A RECOMMENDATION VALUE OF A CONTROL PARAMETER OF A FLUID INFUSION DEVICE**

(57)   A control device (30) for determining a recommendation value of a control parameter of a fluid infusion device (20). The control device (30) comprises a retrieving unit (32) configured to retrieve user data. Each data of the user data having a timestamp and the user data being related to a unique user. The user data comprises at least a plurality of amounts of a drug infused to the unique user; a plurality of physiological values of the unique user; and a plurality of estimated values. The control device (30) also comprises a recommendation unit (34). The recommendation unit (34) is configured to determine the recommendation value based at least on a data of the user data and using a reinforcement learning algorithm comprising a plurality of initial reinforcement parameters.

[Fig. 1]

**Description**

**FIELD OF THE INVENTION**

The present invention relates to a device and method for determining a recommendation value of a control parameter of a fluid infusion device using a reinforcement learning algorithm.

**BACKGROUND OF THE INVENTION**

In the field of healthcare, and more precisely in the field of treating diabetes, it is well known to determine a recommendation value corresponding to an insulin amount in order to maintain the blood glucose, also called glycemia, into a safe range, between hypoglycemia and hyperglycemia, called euglycemia.

Recently, so-called "closed-loop" systems were developed, as described in US20120078067, where a processor is programmed to evaluate a volume rate of insulin to be injected, based on user-related data and/or time-based data, such as past and/or present glycemia measurements, and to control the injection of insulin based on this evaluation. In addition, the processor can be programmed to evaluate a volume of insulin to be injected in some special circumstances, in particular meals, and/or physical activity. The quantity can be injected to the user, subject to the user's approval. Such systems are also called "semi closed-loop" systems because of the necessary declaration by the patient of some of these special circumstances.

The time-based data is often used to predict the future glycemia. This prediction is then used to calculate the quantity of insulin having to be injected in order to maintain the glycemia in acceptable range.

An incorrect prediction of the future blood glucose can lead to an irrelevant calculated quantity of insulin to be injected, leading to a concentration of blood glucose in unacceptable intervals, where the patient may be in hypoglycemia and/or hyperglycemia.

One main drawback of this method is that the method can not precisely take into account all the parameters influencing the general behaviour of the glycemia. This leads to a determination of a recommendation value lacking accuracy.

The invention thus aims to answer at least partially the above presented technical problems.

**BRIEF SUMMARY OF THE INVENTION**

Thus, the invention relates to a control device for determining a recommendation value of a control parameter of a fluid infusion device, the control device comprises:

a retrieving unit, the retrieving unit being configured to retrieve user data, each data of the user data having a timestamp and the user data being related to a unique user, the user data comprising at least:
a plurality of amounts of a drug infused to the unique user;
a plurality of physiological values of the unique user;
a plurality of estimated values;
a recommendation unit, the recommendation unit being configured to determine the recommendation value based at least on a data of the user data and using a reinforcement learning algorithm comprising a plurality of initial reinforcement parameters, wherein the reinforcement learning algorithm is being trained by:
modifying at least an initial reinforcement parameter in order to obtain at least a training reinforcement parameter;
giving at least, as an entry to a parameter calculation method of the reinforcement learning algorithm, at least part of the plurality of amounts of a drug infused to the unique user, at least part of the plurality of physiological values of the unique user and a least part of the plurality of estimated values;
applying the at least one training reinforcement parameter and determining a recommendation value as an exit;
calculating a reward score, the reward score being calculated based at least on the impact of the recommendation value on the plurality of physiological values of the unique user; and
updating at least an initial reinforcement parameter of the plurality of initial reinforcement parameters based on the reward score.

Such a control device allows to determine a recommendation value with more and more accuracy, the more it is used.

Such a control device is extremely flexible and adaptive.

Such a configuration allows to obtain a robust control device without further prior knowledge such as total daily dose, average basal rate in use for a diabetic unique patient for example.

According to an embodiment, the drug infused to the user is insulin.

According to an embodiment, the recommendation value corresponds to a recommended value of insulin. The recommendation value is injected during the training of the reinforcement learning algorithm. According to a specific embodiment, the recommendation unit is configured to not recommend more than sixty units of insulin per hour. The recommendation unit is also configured to not recommend more than three units of insulin per hour during the night. Such a configuration allows to obtain a recommendation unit with a good accuracy as limiting the amount of insulin per hour during the training to specific values allowing to improve the training and the safety of the unique user.

According to the present invention, the terms injected or injection must be understood as a virtual injection in case the training of the reinforcement learning algorithm is made using a simulation wherein the unique user is a virtual user.

According to an embodiment, the control device comprises a converter unit, the converter unit being configured to convert the recommended value of insulin into a control parameter of the fluid infusion device. According to an embodiment, the parameter takes the form of a bolus and/or a basal. Such a configuration allows the infusion device to infuse the recommendation value to the unique user.

According to an embodiment, the plurality of physiological values of the unique user are blood glucose values.

According to an embodiment, the plurality of estimated values are Carbohydrates On Board (COB) values. A COB can be estimated based on blood glucose values and carbohydrates intakes such as meal size for example. A COB represents the carbohydrates ingested by the unique user whose impact cannot yet be measured in the blood glucose.

According to an embodiment, the user data comprise twenty four points of glycemia values, twenty four points of insulin infused to the user and twenty four points of COB. These points represent an input set s. Each point having a timestamp and being separated from the nearest point(s) by substantially five minutes. Substantially five minutes corresponds to the blood glucose measurement rate and can therefore change depending on the measurement method. According to the present invention, substantially five minutes means five minutes plus or minus one minute.

According to an embodiment, the recommendation unit is being configured to determine the recommendation value based on at least one amount of drug infused to the unique user of the plurality of amounts of drugs infused to the user, at least one physiological value of the plurality of physiological values of the unique user and at least one estimated value of the plurality of estimated values of the plurality of estimated values.

According to an embodiment, applying the at least one training reinforcement parameter and determining a recommendation value as an exit means that if there is a total of ten initial reinforcement parameters and only one is modified in order to obtain a training reinforcement parameter, what is applied is nine initial reinforcement parameters and one training reinforcement parameter.

According to an embodiment, the training is iterative. Such a configuration allows the control device to be refined through iterations.

According to an embodiment, each iteration has a time length substantially equal to ten days. Such a configuration allows to take into account long term impact of some parameters or other variables without needing a too long period to train the reinforcement learning algorithm. It is particularly efficient in case of a use of the control device for determining an insulin recommendation value as both insulin and carbohydrates have long term impacts on glycemia. According to the present invention, substantially ten days means ten days, plus or minus one day.

According to an embodiment, the parameter calculation method can be of any type such as a deep neural network - generally called policy network - or a decision tree.

According to an embodiment, the parameter calculation method is a neural network which may be a deep neural network taking as an input the state of the unique user according to the user data and outputting a recommendation value such as an insulin value.

According to an embodiment, the parameter calculation method is a Multi Layer Perceptron (MLP) comprising a first layer, two hidden layers and a last layer. Each hidden layer has sixty-four neurons. The first layer and the two hidden layers each have a tanh activation. Such a configuration allows to maintain a good balance between expressiveness and generalisation of the parameter calculation method and therefore allows to obtain a control device both robust and precise.

It must be noted that any type of parameter calculation method could be used such as a Recurrent Neural Network (RNN), a Gated Recurrent Unit (GRU), a Long Short-Term Memory (LSTM), a Convolution Neural Network (CNN), or a proportional-integral-derivative (PID).

According to an embodiment, the reinforcement learning algorithm is being trained by giving, at least as an entry to a parameter calculation method of the reinforcement learning algorithm, at least part of the plurality of amounts of a drug infused to the unique user, at least part of the plurality of physiological values of the unique user, at least part of the plurality of estimated values and a physiological target. The physiological target represents a physiological value and corresponds to a target that the control device should aim for. The recommendation unit also being configured to determine the recommendation value based on at least an amount of drug infused to the unique user of the plurality of amounts of a drug infused to the unique user, at least a physiological value of the plurality of physiological values of the unique user, at least an estimated value of the plurality of estimated values and the physiological target. The physiological target changes according to the unique user behaviour such as a meal or a physical activity. Such a configuration allows the control device to take into account the unique user behaviour even after the reinforcement learning algorithm training.

According to a specific embodiment, the reinforcement learning algorithm is being trained by giving as an entry to a parameter calculation method of the reinforcement learning algorithm:

several points of glycemia values corresponding to a plurality of physiological values of the unique user;

several points of insulin infused to the user;
several points of COB;
a physiological target; and
an acceptable range;
wherein the physiological target and the acceptable range can vary with time. Such a configuration allows the control device to have a trained reinforcement learning algorithm taking into account the unique user behaviour. The recommendation unit is configured to determine the recommendation value based on several points of glycemia values corresponding to a plurality of physiological values of the unique user, several points of insulin infused to the user, several points of COB, a physiological target, and an acceptable range.

According to an embodiment the physiological target and the acceptable range vary depending on the unique user behaviour.

According to a specific embodiment compatible with the embodiments presented above, the user data also comprise at least one meal announcement. The recommendation unit is therefore being configured to determine the recommendation value based on:

several points of glycemia values corresponding to a plurality of physiological values of the unique user;
several points of insulin infused to the user;
several points of COB; and
at least one meal announcement;
wherein the reinforcement learning algorithm is being trained by giving at least, as an entry to the parameter calculation method, the same data. According to the present invention, a meal announcement represents an announcement of a future meal at a future time having a certain amount of carbohydrates. Such a configuration allows to obtain a control device capable of adjusting its behaviour and improving his meal management. The meal announcement may also comprise a measure of fat content of the meal and/or the time that separates the unique user from the meal.

According to an embodiment, the recommendation unit is configured to determine the recommendation value based at least on a data of the user data and using a plurality of reinforcement learning algorithms. Each reinforcement learning algorithm of the plurality of reinforcement learning algorithms is configured to take at least a data of the user data as an entry and output a recommendation value. In this embodiment, the recommendation unit averages a plurality of outputs from the plurality of reinforcement learning algorithms. Each of the reinforcement learning algorithms is being trained as described above but differs from the other reinforcement learning algorithms at least based on one of the following:

at least an initial reinforcement parameter;
the unique user behaviour during the training, such as sensor noise, or meal patterns for example;
the parameter calculation method;
a length of a training period; and
any other parameter.

Such a configuration allows to obtain a control device determining a recommendation value with good accuracy.

According to an embodiment, the reinforcement learning algorithm is iterative. At each iteration, the recommendation unit is being tested a predefined amount of time. Such a configuration allows to increase the safety of the control device.

According to an embodiment, the updating of at least one initial reinforcement parameter of the plurality of initial reinforcement parameters based on the reward score is done such as the at least one updated initial reinforcement parameter of the plurality of initial reinforcement parameter does not differ by more than a predetermined percentage to the at least one initial reinforcement parameter of the plurality of initial reinforcement parameters. Such a configuration allows to greatly increase the safety of the control device as the at least one initial reinforcement parameter of the plurality of initial reinforcement parameters is modified step by step. The predetermined percentage can be one percent for example.

According to an embodiment, the reinforcement learning algorithm is being trained using a simulated environment and the unique user used during the reinforcement learning algorithm training is a virtual user.

Such a configuration allows to speed up the training process and reduce the human risk.

According to an embodiment, the reinforcement learning algorithm is trained using several virtual users. Such a configuration allows to obtain a robust control device as the reinforcement learning algorithm has been trained in various situations.

According to an embodiment in which the training of the reinforcement learning algorithm is iterative, for each iteration, said reinforcement learning algorithm is being trained on different conditions in order to obtain different reward scores corresponding to the same at least one training reinforcement parameter. The reinforcement learning algorithm is then trained using a different at least one training reinforcement parameter but on the same conditions as before in order to compare the different at least one training reinforcement parameter and therefore obtain a control device more robust and accurate. According to an embodiment, training the reinforcement learning algorithm on three different conditions with the same at least one training reinforcement parameter allows to obtain a robust and accurate control device without needing too much computational load during the training.

According to a specific embodiment in which the reinforcement learning algorithm is being trained by giving at least, as an entry several points of glycemia values corresponding to a plurality of physiological values of the unique user;

several points of insulin infused to the user;
several points of COB; and
at least one meal announcement;
wherein the reinforcement learning algorithm is trained with at least one noisy announcement. Such a configuration allows to obtain a robust control device, especially in case of wrong meal announcement once the reinforcement learning algorithm is trained. The at least one noisy announcement can be of any sort such as:
a meal announcement while the virtual user doesn't ingest any meal;
a meal announcement comprising false informations such as a wrong carbohydrates amounts or a wrong lipids indication; or
no meal announcement while the user ingests a meal for example.

According to an embodiment, the virtual user is based on the unique user. Such a configuration allows to obtain a very precise control device as the reinforcement learning algorithm is specifically trained using a virtual user based on the unique user while still allowing to speed up the training process and reduce the human risk.

According to an embodiment, the reinforcement learning algorithm is being trained by trying different sets of at least one training reinforcement parameter.

According to the present invention, a set of at least one training reinforcement parameter is at least an initial reinforcement parameter modified in order to obtain at least a training reinforcement parameter. Therefore, different sets are different in a way that each set has at least one training reinforcement parameter different from the other sets.

According to an embodiment in which the reinforcement learning algorithm is being trained using a simulated environment, the characteristics of said simulated environment remain the same for each set of at least one reinforcement parameter. Such a configuration allows to calculate a reward score solely based on said set. The characteristics of the simulated environment are for example from the unique user, his behaviours and other random variables for example.

According to an embodiment, the updating is as follow:

$$\theta = \theta + \frac{\varepsilon}{2k\sigma s} \Sigma_{k \in TopDir} \left( F(\theta + e_k) - F(\theta - e_k) \right) e_k$$

wherein:

$\theta$ represents the plurality of initial reinforcement parameters;
e represents the difference between the plurality of initial reinforcement parameters and at least a training reinforcement parameter;
the (e1, ..., ek) are sampled along a normal distribution with variance $\sigma$;
k represents the number of sets of at least one training reinforcement parameters;
s represents the standard deviation of (F(Θ+e1), F(Θ-e1), ..., F(O+ek), F(O-ek));
TopDir represents the best directions, or in other words the ek with highest reward scores obtained by the different sets of at least one training reinforcement parameters;

and $\varepsilon$ represents a learning rate.

Such a configuration allows to obtain a control device with good robustness and adaptability.

According to an embodiment, the learning rate is fixed. Such a configuration allows to control the training with accuracy.

According to an embodiment, the learning rate is exponentially decreasing throughout the iterations in order to "motivate" stronger changes in early iterations and smaller changes in later iterations. Such a configuration allows to avoid overshooting an optimum at each step.

According to an embodiment, s = 30. Such a configuration allows to observe a better convergence behaviour.

According to an embodiment, the user data are normalised.

Such a configuration allows to obtain a faster and more accurate convergence of the reinforcement learning algorithm during training.

According to an embodiment, the user data are modified to comprise noise.

Such a configuration allows to obtain a control device with great robustness.

According to an embodiment, the plurality of amounts of a drug infused to the unique user and the plurality of physiological values of the unique user are modified to comprise noise. Such a configuration allows to obtain a control device with great robustness.

According to an embodiment, the noise can be of any type such as a Gaussian or a specific noise reproducing the noise of a Continuous Glucose Monitor (CGM) for example. Such a configuration allows to obtain a control device with great robustness in particular in its application with a diabetic user using a CGM.

According to an embodiment, the reward score is calculated as follow:

If

$$PHY(n) < THRl, \text{ then } K(n) = (PHY(n) - THRl)^2 + (TAR - THRl)^2$$

If

$$PHY(n) > THRh, \text{ then } K(n) = (PHY(n) - THRh)^2 + (TAR - THRh)^2$$

Else

$$K(n) = (PHY(n) - TAR)^2$$

Then all the K(n) of a determined period of time are summed in order to obtain the reward score.
Wherein:

PHY(n) represents a physiological value of the plurality of physiological values of the unique user with a timestamp n;
THRl represents a lower threshold value of a range;
K(n) represents the reward score at a time n;
THRh represents an higher threshold value of the range; and
TAR represents a physiological target.

Such a configuration allows to calculate the reward score during the whole determined period of time without discount and improve the robustness of the control device in case of long term impact of some parameters or other variables. It is particularly efficient in case of a use of the control device for determining an insulin recommendation value as both insulin and carbohydrates have long term impacts on glycemia.

According to an embodiment, the determined period of time is a period of time corresponding to a period during which the reinforcement learning algorithm is trained.

According to an embodiment in which the control device is used for determining a value of insulin:

PHY(n) represents the blood glucose of the unique user with a timestamp n;
THRl represents the hypoglycemia threshold value of a normoglycemia range; and
THRh represents the hyperglycemia threshold value of the normoglycemia range.

According to an embodiment, the reward score is calculated as follow:

If

$$PHY(n) < THRl, \text{ then } K(n) = \kappa*(PHY(n)-THRl)^2 +(TAR-THRl)^2$$

If

$$PHY(n) > THRh, \text{ then } K(n) = \mu* (PHY(n)-THRh)^2 +(TAR-THRh)^2$$

Else

$$K(n) = (PHY(n)-TAR)^2$$

Then all the K(n) of the determined period of time are summed in order to obtain the reward score.

Wherein:

$\kappa$ represents a tunable parameter; and
$\mu$ represents a tunable parameter.

Such a configuration allows to further optimise the calculation of the reward score by refining the balance between the different glycemia regimes for example.

According to an embodiment, the reward score is calculated based on the blood glucose variation. Such a configuration allows to increase the unique user comfort as the control device tends to reduce the blood glucose variation as much as possible. The reward score is also calculated based on the recommendation values wherein the recommendation values are insulin amounts, the higher the insulin amount during a certain time is, the lower the reward score is. Such a configuration allows to smooth the insulin amount over time and limit the total amount of insulin amounts and therefore reduce the progression of an insulin resistance of the unique user as well as reducing the small blood glucose changes and therefore improve the unique user's health.

According to an embodiment, the reward score is also calculated using penalty coefficients such as hypoglycemia penalty coefficient, hyperglycemia penalty coefficient or target penalty coefficient O for example. Wherein the reward score is calculated as follow:

$$\text{If } THRl < = PHY(n) < = THRh, \text{ then } K(n) = O*(PHY(n)-TAR)^2$$
$$\text{else, if } PHY(n) < THRl, \text{ then }$$
$$K(n) = \Phi* (PHY(n)-THRl)^2 + O*(PHY(n)-TAR)^2$$
$$\text{else } K(n) = M (PHY(n)-THRh)^2 + O*(PHY(n)-TAR)^2$$

Then all the K(n) of the determined period of time are summed in order to obtain the reward score. wherein:

O represents the target penalty coefficient;
$\Phi$ represents the hypoglycemia penalty coefficient; and
M represents the hyperglycemia penalty coefficient.

Such a characteristic allows to more accurately train the reinforcement learning algorithm by modifying the penalty coefficients depending on what matters the most for the unique user. We could, for example, apply a more severe penalty for hypoglycemia than hyperglycemia as hypoglycemia often implies a more important risk to the unique user.

According to an embodiment, the reward score is reduced if PHY(n) is outside an acceptable range.

Such a configuration allows to lower the reward score in specific cases and therefore improve the updating of the at least initial reinforcement parameter of the plurality of reinforcement parameters.

According to an embodiment, if PHY(n) is outside the acceptable range, none of the next points are taken into account. Such a configuration allows to strongly reduce the reward score in certain cases. Indeed, if during the training reinforcement learning algorithm, a simulation simulates a virtual user during a period of ten days, and PHY(n) is outside the acceptable range once during the first day for exemple, none of the points having a timestamp younger that the point wherein PHY(n) is outside the acceptable range will be taken into account for calculating the reward score. Such a configuration allows to obtain a control device determining a recommendation value that has a very low risk of putting PHY(n) outside an acceptable range.

According to an embodiment in which PHY(n) represents the blood glucose of the unique user with a timestamp n, the acceptable range could be between a hypoglycemia and a hyperglycemia respectively corresponding to 70 mg/dL and 180 mg/dL blood glucose.

According to an embodiment, the reward score is reduced if PHY(n) is outside an acceptable range and wherein the reward score is more strongly reduced if PHY(n) is under a lower limit of the acceptable range than above an upper limit of the acceptable range.

According to an embodiment in which PHY(n) represents the blood glucose of the unique user with a timestamp n, the lower limit of the range represents a hypoglycemia level and the upper limit of the range represents a hyperglycemia level. Such a configuration allows to obtain a safer control device as the reinforcement learning algorithm is trained to avoid any hypoglycemia more aggressively than an hyperglycemia.

According to an embodiment, if PHY(n) is under the lower limit of the acceptable range, none of the next points are taken into account while if PHY(n) is above the upper limit of the acceptable range, the next points are taken into account but lowered by a coefficient having a value lower than one. Such a configuration allows to strongly reduce the reward score in certain cases. Indeed, if during the reinforcement learning algorithm training, a simulation simulates a virtual user during a period of ten days, and PHY(n) is under the lower limit of the acceptable range once during the first day for exemple, none of the points having a timestamp younger that the point wherein PHY(n) is outside the acceptable range will be taken into account for calculating the reward score. Such a configuration allows to obtain a control device determining a recommendation value that has a very low risk of putting PHY(n) under the lower limit of the acceptable range and a low risk of putting PHY(n) above the upper limit.

According to an embodiment, the recommendation unit is being configured to determine the recommendation value based on at least a data of the user data, said data having a timestamp corresponding to a period of interest of a certain type.

Such a configuration allows the use of the control device only during periods of interest of a certain type wherein the control device shows the best results.

According to an embodiment, the recommendation unit is being configured to determine the recommendation value based on at least a data of the user data, said data having a timestamp corresponding to a period of interest of a certain type and wherein the reinforcement learning algorithm is being trained by giving at least, as an entry to a parameter calculation method of the reinforcement learning algorithm at least part of the plurality of amounts of a drug infused to the unique user having a timestamp corresponding to a period of interest of the certain type, at least part of the plurality of physiological values of the unique user having a timestamp corresponding to a period of interest of the certain type and a least part of the plurality of estimated values having a timestamp corresponding to a period of interest of the certain type. Such a configuration allows to obtain a control device able to accurately determine a recommendation value based on at least a data of the user data having a timestamp corresponding to a period of interest of a certain type as the reinforcement learning algorithm has been trained using data having a timestamp corresponding to a period of interest of the same certain type. Therefore, the reinforcement learning algorithm is specifically trained for a period of interest of the certain type.

According to an embodiment, several control devices can be used, wherein each control device is in charge of a certain type of period of interest. Such a configuration allows the use of a certain type of control device during a certain type of period of interest that has a reinforcement learning algorithm specifically trained using this certain type of period of interest.

According to an embodiment, there is three certain types of period of interest:

- a meal period type, a meal period is a period which start at the beginning of a carbohydrates intake and end under conditions such as an average deviation between an estimated value of the plurality of estimated values, such as future glycemia, and a physiological value of the plurality of physiological values is only due to insulin and not carbohydrates intakes;
- a resting period type, a resting period is a period during which an estimated value of the plurality of estimated values such as an Insulin On Board (IOB) is low and a physiological value of the plurality of physiological values such as a blood glucose is only influenced by a basal insulin; and
- a filling period type, a filling period is a period that is not a meal period nor a resting period, nor a physical period corresponding to a period of physical activity.

According to an embodiment, the control device also comprises a safety unit, the safety unit being configured to determine if a status of the unique user is at risk, and if so, determine a recommendation value based at least on a data of the user data.

Such a configuration allows the control device to be safer as the recommendation unit is overridden in case a status of the unique user is at risk.

According to an embodiment, a status of the unique user can be of any type such as the blood glucose level for example. In said example, the blood glucose level can be considered at risk outside the range of 70-180 mg/dL or when this value is at a high risk of going below 70 mg/dL in the upcoming hour. This risk may be determined by performing a linear regression on the past glycemia values.

According to an embodiment, the safety unit is also configured to estimate the IOB and adapt the recommendation value in order to limit the risk of hypoglycemia for the unique user.

The invention also relates to a method for determining a recommendation value of a control parameter of a fluid infusion device, the method being implemented by the control device as described above and comprising the steps of:

retrieving user data, each data of the user data having a timestamp and the user data being related to a unique user, the user data comprising at least:
a plurality of amounts of a drug infused to the unique user;
a plurality of physiological values of the unique user;
a plurality of estimated values; and
determining the recommendation value of a control parameter of the fluid infusion device based at least on a data of the user data and using a reinforcement learning algorithm comprising a plurality of initial reinforcement parameters.

According to an embodiment, the method also comprises a preliminary step of training the reinforcement learning algorithm according to the previously described embodiments. The reinforcement learning algorithm can be trained using a control device or any other device.

According to an embodiment, the method may also comprise any step previously described as an embodiment of the control device as said method is implemented, at least partially, by the control device.

According to an embodiment, the training of the reinforcement learning algorithm comprises the steps of:

retrieving user data;
normalising user data;
noising user data so the user data comprise noise;
modifying at least an initial reinforcement parameter in order to obtain at least a training reinforcement parameter;
giving as an entry to a parameter calculation method of the reinforcement learning algorithm, at least part of the plurality of amounts of a drug infused to the unique user, at least part of the plurality of physiological values of the unique user and a least part of the plurality of estimated values;
determining a recommendation value as an exit by applying the at least one training reinforcement parameter ;
calculating a reward score, the reward score being calculated based at least on the impact of the recommendation value on the plurality of physiological values of the unique user; and
updating at least an initial reinforcement parameter of the plurality of initial reinforcement parameters based on the reward score.

Wherein the steps of retrieving user data, normalising user data, noising user data, giving as an entry, and determining a recommendation value are repeated during the determined period, and wherein the steps of retrieving user data, normalising user data, noising user data, giving as an entry, determining a recommendation value, calculating a reward score and updating at least an initial reinforcement parameter are repeated a determined number of times. Such a configuration allows to train a robust and accurate reinforcement learning algorithm.

The invention also relates to a computer program product comprising instructions which, when the program is executed by a computer, cause the computer to carry out the steps of the method as described above.

The various non-incompatible aspects defined above can be combined.

## BRIEF DESCRIPTION OF THE DRAWINGS

Embodiments of the invention will be described below with reference to the drawings, described briefly below:

[Fig.1] shows a schematic view of a control device according to one embodiment of the invention; and
[Fig.2] shows steps of a method for determining the recommendation value of a control parameter of the fluid infusion device according to one embodiment of the invention.

**DETAILED DESCRIPTION OF THE INVENTION**

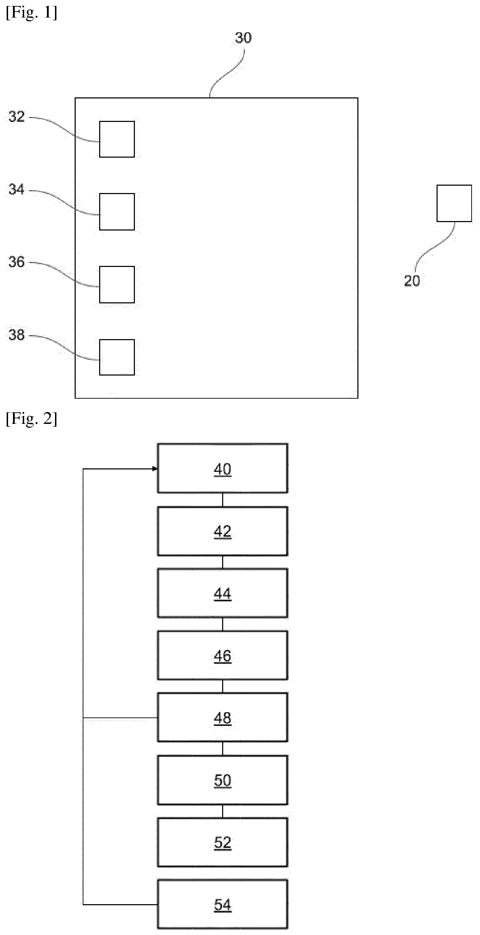

As shown in [Fig.1], the present invention relates to a control device 30 for determining a recommendation value, corresponding to a recommended value of insulin, of a control parameter of a fluid infusion device 20. The control device 30 comprises a retrieving unit 32 configured to retrieve user data, each data of the user data having a timestamp and the user data being related to a unique user and comprising noise. Such a configuration allows to obtain a control device 30 with great robustness. The noise can be of any type such as a Gaussian or a specific noise reproducing the noise of a Continuous Glucose Monitor (CGM) for example. Such a configuration allows to obtain a control device 30 with great robustness in particular in its application with a diabetic user using a CGM.

The user data comprises at least:

a plurality of amounts of a drug infused to the unique user, the amount of drug infused correspond to insulin;
a plurality of physiological values of the unique user, the physiological values of the unique user correspond to blood glucose values; and
a plurality of estimated values, the estimated values correspond to Carbohydrates On Board (COB) values.

A COB can be estimated based on blood glucose values and carbohydrates intakes such as meal size for example. A COB represents the carbohydrates ingested by the unique user whose impact cannot yet be measured in the blood glucose.

The control device 30 also comprises a recommendation unit 34 configured to determine the recommendation value based at least on a data of the user data and using a reinforcement learning algorithm comprising a plurality of initial reinforcement parameters. More specifically, the Recommendation unit 34 is being configured to determine the recommendation value based on at least one amount of insulin infused to the unique user of the plurality of amounts of drugs infused to the user, at least one blood glucose value of the plurality of physiological values of the unique user and at least one COB of the plurality of estimated values of the plurality of estimated values.

The control device 30 comprises a converter unit 38 configured to convert the recommended value of insulin into a control parameter of the fluid infusion device 20. According to an embodiment, the parameter takes the form of a bolus and/or a basal. Such a configuration allows the infusion device 20 to infuse the recommendation value to the unique user.

The reinforcement learning algorithm is being trained by:

modifying at least an initial reinforcement parameter in order to obtain at least a training reinforcement parameter;
giving at least, as an entry to a parameter calculation method, of any type such as a deep neural network also called a policy network or a decision tree, of the reinforcement learning algorithm, at least part of the plurality of amounts of a drug infused to the unique user, at least part of the plurality of physiological values of the unique user and a least part of the plurality of estimated values;
applying the at least one training reinforcement parameter and determining a recommendation value as an exit;
calculating a reward score, the reward score being calculated based at least on the impact of the recommendation value on the plurality of physiological values of the unique user; and
updating at least an initial reinforcement parameter of the plurality of initial reinforcement parameters based on the reward score.

According to an embodiment, applying the at least one training reinforcement parameter and determining a recommendation value as an exit means that if there is a total of ten initial reinforcement parameters and only one is modified in order to obtain a training reinforcement parameter, what is applied is nine initial reinforcement parameters and one training reinforcement parameter. The parameter calculation method is a Multi Layer Perceptron (MLP) comprising a first layer, two hidden layers and a last layer. Each hidden layer has sixty-four neurons. The first layer and the two hidden layers each have a tanh activation. Such a configuration allows to maintain a good balance between expressiveness and generalisation of the parameter calculation method and therefore allows to obtain a control device 30 both robust and precise.

Such a control device 30 allows to determine a recommendation value with more and more accuracy, the more it is used. Such a control device 30 is also extremely flexible and adaptive. Such a configuration also allows to obtain a robust control device 30 without further prior knowledge such as total daily dose, average basal rate in use for a diabetic unique patient for example.

According to a specific embodiment, the Recommendation unit 34 is configured to not recommend more than sixty units of insulin per hour. The Recommendation unit 34 is also configured to not recommend more than three units of insulin per hour during the night. Such a configuration allows to obtain a Recommendation unit 34 with a good accuracy as limiting the amount of insulin per hour during the training to specific values allowing to improve the training and the safety of the unique user.

According to an embodiment, the user data comprise twenty four points of glycemia values, twenty four points of insulin infused to the user and twenty four points of COB. These points represent an input set s. Each point having a timestamp and being separated from the nearest point(s) by substantially five minutes. Substantially five minutes corresponds to the blood glucose measurement rate and can therefore change depending on the measurement method. According to the present invention, substantially five minutes means five minutes plus or minus one minute.

According to an embodiment, the reinforcement learning algorithm is being trained by giving, at least as an entry to a parameter calculation method of the reinforcement learning algorithm, at least part of the plurality of amounts of a drug infused to the unique user, at least part of the plurality of physiological values of the unique user, at least part of the plurality of estimated values and a physiological target. The physiological target represents a physiological value and corresponds to a target that the control device 30 should aim for. The Recommendation unit 34 also being configured to determine the recommendation value based on at least an amount of drug infused to the unique user of the plurality of amounts of a drug infused to the unique user, at least a physiological value of the plurality of physiological values of the unique user, at least an estimated value of the plurality of estimated values and the physiological target. The physiological target changes according to the unique user behaviour such as a meal or a physical activity. Such a configuration allows the control device 30 to take into account the unique user behaviour even after the reinforcement learning algorithm training.

According to a specific embodiment, the reinforcement learning algorithm is being trained by giving as an entry to a parameter calculation method of the reinforcement learning algorithm:

several points of glycemia values corresponding to a plurality of physiological values of the unique user;
several points of insulin infused to the user;
several points of COB;
a physiological target; and
an acceptable range;
wherein the physiological target and the acceptable range can vary with time. Such a configuration allows the control device 30 to have a trained reinforcement learning algorithm taking into account the unique user behaviour. The Recommendation unit 34 is configured to determine the recommendation value based on several points of glycemia values corresponding to a plurality of physiological values of the unique user, several points of insulin infused to the user, several points of COB, a physiological target, and an acceptable range. The physiological target and the acceptable range vary depending on the unique user behaviour.

The accuracy of the control device 30 may also be improved if the user data also comprise at least one meal announcement. The Recommendation unit 34 is therefore being configured to determine the recommendation value based on:

several points of glycemia values corresponding to a plurality of physiological values of the unique user;
several points of insulin infused to the user;
several points of COB; and
at least one meal announcement;
wherein the reinforcement learning algorithm is being trained by giving at least, as an entry to the parameter calculation method, the same data. According to the present invention, a meal announcement represents an announcement of a future meal at a future time having a certain amount of carbohydrates. Such a configuration allows to obtain a control device 30 capable of adjusting its behaviour and improving his meal management. The meal announcement may also comprise a measure of fat content of the meal and/or the time that separates the unique user from the meal.

Another way to greatly improve the accuracy of the control device 30 is to have a Recommendation unit 34 configured to determine the recommendation value based at least on a data of the user data and using a plurality of reinforcement learning algorithms. Each reinforcement learning algorithm of the plurality of reinforcement learning algorithms is configured to take at least a data of the user data as an entry and output a recommendation value. In this embodiment, the Recommendation unit 34 averages a plurality of outputs from the plurality of reinforcement learning algorithms. Each of the reinforcement learning algorithms is being trained as described above but differs from the other reinforcement learning algorithms at least based on one of the following:

at least an initial reinforcement parameter;
the unique user behaviour during the training, such as sensor noise, or meal patterns for example;
the parameter calculation method;

a length of a training period; and
any other parameter.

Such a configuration allows to obtain a control device 30 determining a recommendation value with good accuracy.

The updating of at least one initial reinforcement parameter of the plurality of initial reinforcement parameters based on the reward score is done such as the at least one updated initial reinforcement parameter of the plurality of initial reinforcement parameter does not differ by more than a predetermined percentage to the at least one initial reinforcement parameter of the plurality of initial reinforcement parameters. Such a configuration allows to greatly increase the safety of the control device 30 as the at least one initial reinforcement parameter of the plurality of initial reinforcement parameters is modified step by step. A preferred predetermined percentage is one percent.

The reinforcement learning algorithm is being trained using a simulated environment and the unique user used during the reinforcement learning algorithm training is a virtual user preferably based on the unique user, or a real user. Such a configuration allows to speed up the training process and reduce the human risk. Such a configuration allows to obtain a very precise control device 30 as the reinforcement learning algorithm is specifically trained using a virtual user based on the unique user while still allowing to speed up the training process and reduce the human risk.

The reinforcement learning algorithm is trained using several virtual users. Such a configuration allows to obtain a robust control device 30 as the reinforcement learning algorithm has been trained in various situations.

According to an embodiment, the training is iterative, and is done as follows:

Start with a policy network $\pi_0$ with initial parameters $\theta$

For i in iterations:

Sample n vectors of noise $(e_1,...,e_n)$ of the same shape as $\theta$ along a normal distribution $N(0, \sigma)$

Sample m random seeds $(s_1.. ., s_m)$ , said random seed are defining all environment randomness

For sign in [-1, 1]: , to run in parallel

For each vector $e_i$: , allows us to evaluate the perfs of this noise

For each random seed $s_j$: Create a virtual patient from random seed $s_j$

Run the simulation using the control provided by the policy network with parameters $\theta$ + sign* $e_i$, until the patient dies or the maximum number of steps is reached. At each time step t, store the reward r(i,j,t)

Compute the total episode reward $R(i,j) = \sum_t r(i,j,t)$

Compute the average reward for the i-th agent $F(i, sign) = 1/m * \sum_j R(i,j)$

Keep only the top k indices in [1, 2, ..., n] sorted by max(F(i, 1), F(i,-1))

Compute s = $std_{hin\ topdir}$ (F(h,1), F(h,-1)) , correcting update by std of evaluations

Update the main parameters using the formula: , allows to approximate gradient step

$$\Theta = \Theta + \varepsilon/(2k\sigma s)\Sigma_{kin\ topdir}(F(k,1) - F(k,-1))e_k$$

Output: the final policy network $\pi_0$

Such a configuration allows the control device 30 to be refined through iterations and increases the safety of the control device 30.

According to an embodiment, each iteration has a time length substantially equal to ten days. Such a configuration allows to take into account long term impact of some parameters or other variables without needing a too long period to train the reinforcement learning algorithm. It is particularly efficient in case of a use of the control device 30 for determining an insulin recommendation value as both insulin and carbohydrates have long term impacts on glycemia. According to the present invention, substantially ten days means ten days, plus or minus one day.

For each iteration, the reinforcement learning algorithm is being trained on different conditions in order to obtain different reward scores corresponding to the same at least one training reinforcement parameter. The reinforcement learning algorithm is then trained using a different at least one training reinforcement parameter but on the same conditions as before in order to compare the different at least one training reinforcement parameter and therefore obtain a control device 30 more robust and accurate. Training the reinforcement learning algorithm on three different conditions with the same at least one training reinforcement parameter allows to obtain a robust and accurate control device 30 without needing too much computational load during the training.

According to a specific embodiment in which the reinforcement learning algorithm is being trained by giving at least, as an entry several points of glycemia values corresponding to a plurality of physiological values of the unique user;

several points of insulin infused to the user;
several points of COB; and
at least one meal announcement;
wherein the reinforcement learning algorithm is trained with at least one noisy announcement. Such a configuration allows to obtain a robust control device 30, especially in case of wrong meal announcement once the reinforcement learning algorithm is trained. The at least one noisy announcement can be of any sort such as:
a meal announcement while the virtual user doesn't ingest any meal;
a meal announcement comprising false informations such as a wrong carbohydrates amounts or a wrong lipids indication; or
no meal announcement while the user ingests a meal for example.

According to a specific embodiment, the reinforcement learning algorithm is being trained by trying different sets of at least one training reinforcement parameter. A set of at least one training reinforcement parameter is at least an initial reinforcement parameter modified in order to obtain at least a training reinforcement parameter. Therefore, different sets are different in a way that each set has at least one training reinforcement parameter different from the other sets.

To calculate a reward score solely based on a set, the characteristics of the simulated environment remain the same for each set of at least one reinforcement parameter. The characteristics of the simulated environment are for example from the unique user, his behaviours and other random variables for example and can also be called "seed".

the updating of the at least an initial reinforcement parameter is as follow:

$$\theta = \theta + \frac{\varepsilon}{2k\sigma s} \Sigma_{k\epsilon TopDir} \left( F(\theta + e_k) - F(\theta - e_k) \right) e_k$$

wherein:

$\theta$ represents the plurality of initial reinforcement parameters;
e represents the difference between the plurality of initial reinforcement parameters and at least a training reinforcement parameter;
the (e1, ..., ek) are sampled along a normal distribution with variance $\sigma$;
k represents the number of sets of at least one training reinforcement parameters;
s represents the standard deviation of (F($\Theta$+e1), F($\Theta$-e1), ..., F(O+ek), F(O-ek));
TopDir represents the best directions, or in other words the ek with highest reward scores obtained by the different sets of at least one training reinforcement parameters; and
and $\varepsilon$ represents a learning rate;

Such a configuration allows to obtain a control device 30 with good robustness and adaptability.

The learning rate can be either fixed to control the training with accuracy or exponentially decreasing throughout the iterations in order to "motivate" stronger changes in early iterations and smaller changes in later iterations. Such a configuration allows to avoid overshooting an optimum at each step.

According to an embodiment, s = 30. Such a configuration allows to observe a better convergence behaviour.

The user data are normalised, such a configuration allows to obtain a faster and more accurate convergence of the reinforcement learning algorithm during training.

The reward score is calculated as follow:
If

$$PHY(n) < THRl, \text{ then } K(n) = (PHY(n) - THRl)^2 + (TAR - THRl)^2$$

If

$$PHY(n) > THRh, \text{ then } K(n) = (PHY(n) - THRh)^2 + (TAR - THRh)^2$$

Else

$$K(n) = (PHY(n) - TAR)^2$$

Then all the K(n) of a determined period of time are summed in order to obtain the reward score. The determined period of time is a period of time corresponding to a period during which the reinforcement learning algorithm is trained. This period had a length of ten days.

Wherein:

PHY(n) represents the blood glucose of the unique user with a timestamp n;
THRl represents the hypoglycemia threshold value of a normoglycemia range;
K(n) represents the reward score at a time n;
THRh represents the hyperglycemia threshold value of the normoglycemia range.
TAR represents a physiological target.

Such a configuration allows to calculate the reward score during the whole determined period of time without discount and improve the robustness of the control device 30 in case of long term impact of some parameters or other variables. It is particularly efficient in case of a use of the control device 30 for determining an insulin recommendation value as both insulin and carbohydrates have long term impacts on glycemia.

According to a specific embodiment, the reward score is calculated as follow:

If

$$PHY(n) < THRl, \text{ then } K(n) = \kappa * (PHY(n) - THRl)^2 + (TAR - THRl)^2$$

If

$$PHY(n) > THRh, \text{ then } K(n) = \mu * (PHY(n) - THRh)^2 + (TAR - THRh)^2$$

Else

$$K(n) = (PHY(n) - TAR)^2$$

Then all the K(n) of the determined period of time are summed in order to obtain the reward score.

Wherein:

$\kappa$ represents a tunable parameter; and
$\mu$ represents a tunable parameter.

Such a configuration allows to further optimise the calculation of the reward score by refining the balance between the different glycemia regimes for example.

The reward score can also be calculated based on the blood glucose variation. Such a configuration allows to increase the unique user comfort as the control device 30 tends to reduce the blood glucose variation as much as possible. The reward score is also calculated based on the recommendation values wherein the recommendation values are insulin amounts, the higher the insulin amount during a certain time is, the lower the reward score is. Such a configuration allows to smooth the insulin amount over time and limit the total amount of insulin amounts and therefore reduce the progression of an insulin resistance of the unique user as well as reducing the small blood glucose changes and therefore improve the unique user's health.

The reward score can also be calculated using penalty coefficients such as hypoglycemia penalty coefficient, hyperglycemia penalty coefficient or target penalty coefficient O for example. Wherein the reward score is calculated as follow:

$$\text{If } THRl <= PHY(n) <= THRh, \text{ then } K(n) = O*(PHY(n)-TAR)^2$$
$$\text{else, if } PHY(n) < THRl, \text{ then}$$
$$K(n) = \Phi*(PHY(n)-THRl)^2 + O*(PHY(n)-TAR)^2$$
$$\text{else } K(n) = M(PHY(n)-THRh)^2 + O*(PHY(n)-TAR)^2$$

Then all the K(n) of the determined period of time are summed in order to obtain the reward score. wherein:

> O represents the target penalty coefficient;
> $\Phi$ represents the hypoglycemia penalty coefficient; and
> M represents the hyperglycemia penalty coefficient.

Such a characteristic allows to more accurately train the reinforcement learning algorithm by modifying the penalty coefficients depending on what matters the most for the unique user. We could, for example, apply a more severe penalty for hypoglycemia than hyperglycemia as hypoglycemia often implies a more important risk to the unique user.

Lowering the score in specific cases also improves the updating of the at least initial reinforcement parameter of the plurality of reinforcement parameters and therefore improves the safety of the control device 30 when the reward score is reduced if PHY(n) is outside an acceptable range for example. According to an embodiment in which PHY(n) represents the blood glucose of the unique user with a timestamp n, the acceptable range could be between a hypoglycemia and a hyperglycemia respectively corresponding to 70 mg/dL and 180 mg/dL blood glucose.

According to a specific embodiment, if PHY(n) is outside the acceptable range, none of the next points are taken into account. Such a configuration allows to strongly reduce the reward score in certain cases. Indeed, if during the training reinforcement learning algorithm, a simulation simulates a virtual user during a period of ten days, and PHY(n) is outside the acceptable range once during the first day for exemple, none of the points having a timestamp younger that the point wherein PHY(n) is outside the acceptable range will be taken into account for calculating the reward score. Such a configuration allows to obtain a control device 30 determining a recommendation value that has a very low risk of putting PHY(n) outside an acceptable range.

It is also interesting to have a reduced reward score if PHY(n) is outside an acceptable range and to reduce said reward score even more when PHY(n) is under a lower limit of the acceptable range than above an upper limit of the acceptable range, wherein the upper limit correspond to hyperglycemia and the lower limit correspond to hypoglycemia. Such a configuration allows to increase the safety of the control device 30 as hypoglycemia is often more dangerous than hyperglycemia. In other words, according to an embodiment in which PHY(n) represents the blood glucose of the unique user with a timestamp n, the lower limit of the range represents a hypoglycemia level and the upper limit of the range represents a hyperglycemia level. Such a configuration allows to obtain a safer control device 30 as the reinforcement learning algorithm is trained to avoid any hypoglycemia more aggressively than an hyperglycemia. It is also possible to improve the safety of the control device 30 as if PHY(n) is under the lower limit of the acceptable range, none of the next points are taken into account while if PHY(n) is above the upper limit of the acceptable range, the next points are taken into account but lowered by a coefficient having a value lower than one. Such a configuration allows to strongly reduce the reward score in certain cases. Indeed, if during the reinforcement learning algorithm training, a simulation simulates a virtual user during a period of ten days, and PHY(n) is under the lower limit of the acceptable range once during the first day for exemple, none of the points having a timestamp younger that the point wherein PHY(n) is outside the acceptable range will be taken into account for calculating the reward score. Such a configuration allows to obtain a control device 30 determining a recommendation value that has a very low risk of putting PHY(n) under the lower limit of the acceptable range and a low risk of putting PHY(n) above the upper limit.

The recommendation unit 34 is being configured to determine the recommendation value based on at least a data of the user data, said data having a timestamp corresponding to a period of interest of a certain type. Such a configuration allows the use of the control device 30 only during periods of interest of a certain type wherein the control device 30 shows the best results. More precisely, the Recommendation unit 34 is being configured to determine the recommendation value based on at least a data of the user data, said data having a timestamp corresponding to a period of interest of a certain type and wherein the reinforcement learning algorithm is being trained by giving at least, as an entry to a parameter calculation method of the reinforcement learning algorithm at least part of the plurality of amounts of a drug infused to the unique user having a timestamp corresponding to a period of interest of the certain type, at least part of the plurality of physiological values of the unique user having a timestamp corresponding to a period of interest of the certain type and a least part of the plurality of estimated values having a timestamp corresponding to a period of interest of the certain type. Such a configuration allows to obtain a control device 30 able to accurately determine a recommendation value based on at least a data of the user data having a timestamp corresponding to a period of interest of a certain type as the reinforcement learning algorithm has been trained using data having a timestamp corresponding to a period of interest of the same certain type. Therefore, the reinforcement learning algorithm is specifically trained for a period of interest of the certain type. Therefore, it is possible to use several control devices 30, wherein each control device 30 is in charge of a certain type of period of interest. Such a configuration allows the use of a certain type of control device 30 during a certain type of period of interest that has a reinforcement learning algorithm specifically trained using this certain type of period of interest.

There is three certain types of period of interest:

- a meal period type, a meal period is a period which start at the beginning of a carbohydrates intake and end under conditions such as an average deviation between an estimated value of the plurality of estimated values, such as future glycemia, and a physiological value of the plurality of physiological values is only due to insulin and not carbohydrates intakes;
- a resting period type, a resting period is a period during which an estimated value of the plurality of estimated values such as an Insulin On Board (IOB) is low and a physiological value of the plurality of physiological values such as a blood glucose is only influenced by a basal insulin; and
- a filling period type, a filling period is a period that is not a meal period nor a resting period, nor a physical period corresponding to a period of physical activity.

The control device 30 also comprises a safety unit 36, the safety unit 36 being configured to determine if a status of the unique user is at risk, and if so, determine a recommendation value based at least on a data of the user data. Such a configuration allows the control device 30 to be safer as the Recommendation unit 34 is overridden in case a status of the unique user is at risk. The status of the unique user corresponds to the blood glucose level of the unique user. The blood glucose level can be considered at risk outside the range of 70-180 mg/dL or when this value is at a high risk of going below 70 mg/dL in the upcoming hour. This risk may be determined by performing a linear regression on the past glycemia values. The safety unit 36 is also configured to estimate the IOB and adapt the recommendation value in order to limit the risk of hypoglycemia for the unique user.

As shown in [Fig.2], the present invention also relates to a method for determining a recommendation value of a control parameter of a fluid infusion device 20, the method being implemented by the control device 30 according to the characteristics described above. The method comprises the steps of:

retrieving user data 40, each data of the user data having a timestamp and the user data being related to a unique user, the user data comprising at least:
a plurality of amounts of a drug infused to the unique user;
a plurality of physiological values of the unique user;
a plurality of estimated values; and
determining the recommendation value of a control parameter of the fluid infusion device 20 based at least on a data of the user data and using a reinforcement learning algorithm comprising a plurality of initial reinforcement parameters.

The method also comprises a preliminary step of training the reinforcement learning algorithm according to the previously described embodiments. The reinforcement learning algorithm can be trained using a control device 30 or any other device. The method may also comprise any step previously described as an embodiment of the control device 30 as said method is implemented, at least partially, by the control device 30.

The training of the reinforcement learning algorithm comprises the steps of:

retrieving user data 40;
normalising user data 42;
noising user data 44 so the user data comprise noise;
modifying at least an initial reinforcement parameter 46 in order to obtain at least a training reinforcement parameter;
giving as an entry 48 to a parameter calculation method of the reinforcement learning algorithm, at least part of the plurality of amounts of a drug infused to the unique user, at least part of the plurality of physiological values of the unique user and a least part of the plurality of estimated values;
determining a recommendation value 50 as an exit by applying the at least one training reinforcement parameter;
calculating a reward score 52, the reward score being calculated based at least on the impact of the recommendation value on the plurality of physiological values of the unique user; and
updating at least an initial reinforcement parameter 54 of the plurality of initial reinforcement parameters based on the reward score.

Wherein the steps of retrieving user data 40, normalising user data 42, noising user data 44, giving as an entry 48, and determining a recommendation value 50 are repeated during the determined period, and wherein the steps of retrieving user data 40, normalising user data 42, noising user data 44, giving as an entry 48, determining a recommendation value 50, calculating a reward score 52 and updating at least an initial reinforcement parameter 54 are repeated a determined number of times. Such a configuration allows to train a robust and accurate reinforcement learning algorithm.

The present invention also relates to a computer program product comprising instructions which, when the program is executed by a computer, cause the computer to carry out the steps of the method described above.

While exemplary embodiments of the invention have been described, it will be understood by those skilled in the art that various changes, omissions and/or additions may be made, and equivalents may be substituted for elements thereof without departing from the spirit and scope of the invention. In addition, many modifications may be made to adapt a particular situation or material to the teachings of the invention without departing from the scope thereof. Therefore, it is intended that the invention is not limited to the particular embodiments disclosed for carrying out this invention, but that the invention will include all embodiments falling within the scope of the appended claims. Moreover, unless specifically stated any use of the terms first, second, etc. do not denote any order or importance, but rather the terms first, second, etc. are used to distinguish one element from another.

## Claims

1. A control device (30) for determining a recommendation value of a control parameter of a fluid infusion device (20), the control device (30) comprises:
   a retrieving unit (32), the retrieving unit (32) being configured to retrieve user data, each data of the user data having a timestamp and the user data being related to a unique user, the user data comprising at least:

   a plurality of amounts of a drug infused to the unique user;
   a plurality of physiological values of the unique user;
   a plurality of estimated values;
   a recommendation unit (34), the recommendation unit (34) being configured to determine the recommendation value based at least on a data of the user data and using a reinforcement learning algorithm comprising a plurality of initial reinforcement parameters, wherein the reinforcement learning algorithm is being trained by:

   modifying at least an initial reinforcement parameter in order to obtain at least a training reinforcement parameter;
   giving at least, as an entry to a parameter calculation method of the reinforcement learning algorithm, at least part of the plurality of amounts of a drug infused to the unique user, at least part of the plurality of physiological values of the unique user and a least part of the plurality of estimated values;
   applying the at least one training reinforcement parameter and determining a recommendation value as an exit;
   calculating a reward score, the reward score being calculated based at least on the impact of the recommendation value on the plurality of physiological values of the unique user; and
   updating at least an initial reinforcement parameter of the plurality of initial reinforcement parameters based on the reward score.

2. A control device (30) according to claim 1, wherein the reinforcement learning algorithm is being trained using a simulated environment and the unique user used during the reinforcement learning algorithm training is a virtual user.

3. A control device (30) according to claim 2, wherein the virtual user is based on the unique user.

4. A control device (30) according to any of the claims 1 to 3, wherein the reinforcement learning algorithm is being trained by trying different sets of at least one training reinforcement parameters.

5. A control device (30) according to claim 4, wherein the updating is as follow:

$$\theta = \theta + \frac{\varepsilon}{2k\sigma s} \Sigma_{k \in TopDir} \left( F(\theta + e_k) - F(\theta - e_k) \right) e_k$$

wherein:

$\theta$ represents the plurality of initial reinforcement parameters;

e represents the difference between the plurality of initial reinforcement parameters and at least a training reinforcement parameter;

the $(e_1, ..., e_k)$ are sampled along a normal distribution with variance $\sigma$; k represents the number of sets of at least one training reinforcement parameters;

s represents the standard deviation of $(F(\Theta+e1), F(\Theta-e1), ..., F(O+ek), F(O-ek))$;

TopDir represents the best directions, or in other words the $e_k$ with highest reward scores obtained by the different sets of at least one training reinforcement parameters;

and $\varepsilon$ represents a learning rate.

6. A control device (30) according to any of the claims 1 to 5, wherein the user data are normalised.

7. A control device (30) according to any of the claims 1 to 6, wherein the user data are modified to comprise noise.

8. A control device (30) according to any of the claims 1 to 7, wherein the reward score is calculated as follow:

$$\text{If } PHY(n) < THRl, \text{ then}$$
$$K(n) = (PHY(n) - THRl)^2 + (TAR - THRl)^2$$

$$\text{If } PHY(n) > THRh, \text{ then}$$
$$K(n) = (PHY(n) - THRh)^2 + (TAR - THRh)^2$$

Else

$$K(n) = (PHY(n) - TAR)^2$$

Then all the K(n) of a determined period of time are summed in order to obtain the reward score.
Wherein:

PHY(n) represents a physiological value of the plurality of physiological values of the unique user with a timestamp n;

THRl represents a lower threshold value of a range; K(n) represents the reward score at a time n;

THRh represents an higher threshold value of the range; and TAR represents a physiological target.

9. A control device (30) according to claim 8, wherein the reward score is reduced if PHY(n) is outside an acceptable range.

10. A control device (30) according to claim 9, wherein the reward score is reduced if PHY(n) is outside an acceptable range and wherein the reward score is more strongly reduced if PHY(n) is under a lower limit of the acceptable range than above an upper limit of the acceptable range.

11. A control device (30) according to any of the claims 1 to 10, wherein the recommendation unit (34) is being configured to determine the recommendation value based on at least a data of the user data, said data having a timestamp corresponding to a period of interest of a certain type.

12. A control device (30) according to any of the claims 1 to 11, wherein the control device also comprises a safety unit (36), the safety unit (36) being configured to determine if a status of the unique user is at risk, and if so, determine a recommendation value based at least on a data of the user data.

13. Method for determining a recommendation value of a control parameter of a fluid infusion device (20), the method being implemented by a control device (30) according to any of the claims 1 to 12 and comprising the steps of:

retrieving user data (40), each data of the user data having a timestamp and the user data being related to a unique user, the user data comprising at least:

a plurality of amounts of a drug infused to the unique user;
a plurality of physiological values of the unique user;
a plurality of estimated values; and
determining the recommendation value of a control parameter of the fluid infusion device (20) based at least on a data of the user data and using a reinforcement learning algorithm comprising a plurality of initial reinforcement parameters.

14. A computer program product comprising instructions which, when the program is executed by a computer, cause the computer to carry out the steps of the method of claim 13.

[Fig. 1]

[Fig. 2]

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/235618 A1 (THE US GOV AS REPRESENTED BY THE DEPARTMENT OF VETERANS AFFAIRS [US] E) 10 November 2022 (2022-11-10) | 1-14 | INV. G16H20/17 G06N20/00 G16H40/60 |
| Y | * paragraph [0010] * * paragraph [0080] * * paragraph [0086] - paragraph [0087] * * paragraph [0117] * * paragraph [0186] * * paragraph [0217] * * paragraph [0271] * * paragraph [0275] * ----- | 1-14 | G16H40/63 G16H40/67 G16H50/70 |
| X | CN 111 048 178 A (HANGZHOU ZHISHENG DATA TECH CO LTD) 21 April 2020 (2020-04-21) | 1-14 | |
| Y | * paragraph [0012] - paragraph [0014] * * paragraph [0071] * * paragraph [0167] * ----- | 1-14 | |
| A | LEE SEUNGHYUN ET AL: "Toward a Fully Automated Artificial Pancreas System Using a Bioinspired Reinforcement Learning Design: In Silico Validation", IEEE JOURNAL OF BIOMEDICAL AND HEALTH INFORMATICS, IEEE, PISCATAWAY, NJ, USA, vol. 25, no. 2, 12 June 2020 (2020-06-12), pages 536-546, XP011836212, ISSN: 2168-2194, DOI: 10.1109/JBHI.2020.3002022 [retrieved on 2021-02-04] * the whole document * ----- -/-- | 1-14 | |

**TECHNICAL FIELDS SEARCHED (IPC)**

G16H
G06N

The present search report has been drawn up for all claims

| Place of search | Date of completion of the search | Examiner |
|---|---|---|
| Munich | 11 March 2024 | Rivera Farina, P |

EPO FORM 1503 03.82 (P04C01)

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons
.........................................................................
& : member of the same patent family, corresponding
 document

page 1 of 2

**EUROPEAN SEARCH REPORT**

Application Number

EP 23 20 6115

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WANG ZIHAO ET AL: "Reinforcement Learning-Based Insulin Injection Time And Dosages Optimization", 2021 INTERNATIONAL JOINT CONFERENCE ON NEURAL NETWORKS (IJCNN), IEEE, 18 July 2021 (2021-07-18), pages 1-8, XP033974035, DOI: 10.1109/IJCNN52387.2021.9533957 * the whole document * | 1-14 | |
| A | MARIANO DE PAULA ET AL: "On-line policy learning and adaptation for real-time personalization of an artificial pancreas", EXPERT SYSTEMS WITH APPLICATIONS, vol. 42, no. 4, 1 March 2015 (2015-03-01), pages 2234-2255, XP055418564, AMSTERDAM, NL ISSN: 0957-4174, DOI: 10.1016/j.eswa.2014.10.038 * the whole document * | 1-14 | |

TECHNICAL FIELDS SEARCHED (IPC)

The present search report has been drawn up for all claims

| Place of search | Date of completion of the search | Examiner |
|---|---|---|
| Munich | 11 March 2024 | Rivera Farina, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 23 20 6115

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-2024

| Patent document cited in search report | Publication date | Patent family member(s) | Publication date |
|---|---|---|---|
| WO 2022235618 A1 | 10-11-2022 | NONE | |
| CN 111048178 A | 21-04-2020 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120078067 A